# FASCICULE DE BREVET EUROPEEN

(11) **EP 1 233 711 B1**
(45) Date de publication et mention de la délivrance du brevet: **23.08.2006**
(21) Numéro de dépôt: 00985390.4
(22) Date de dépôt: 01.12.2000
(51) Int. Cl.: A61B 17/70

(54) **DISPOSITIF DE STABILISATION INTERVERTEBRAL**
VORRICHTUNG ZUR ZWISCHENWIRBELSTABILISIERUNG
INTERVERTEBRAL STABILISING DEVICE

(30) Priorité: 01.12.1999 FR 9915160; 29.12.1999 FR 9916662; 24.05.2000 FR 0006640
(43) Date de publication de la demande: 28.08.2002
(73) Titulaire: Graf, Henry, F-69006 Lyon (FR)
(72) Inventeur: Graf, Henry, F-69006 Lyon (FR)
(74) Mandataire: Jacobson, Claude
(86) Numéro de dépôt international: PCT/FR2000/003368
(87) Numéro de publication internationale: WO 2001/039678

(56) Documents cités:
- EP-A- 0 282 161
- EP-A- 0 820 731
- FR-A- 2 676 911
- FR-A- 2 744 010
- GB-A- 1 306 660

## Description

La présente invention concerne un dispositif de stabilisation intervertébral.

De manière habituelle, un tel dispositif est destiné à remplacer tout ou partie d'un disque intervertébral, lorsque ce dernier a été détruit par la chirurgie ou la maladie.

EP-A-282 161 montre un élément de remplacement du disque intervertébral endommagé.

FR-A-2 676 911 montre un amortisseur relié à deux implants vertébral.>

L'invention se propose de réaliser un dispositif de stabilisation qui, tout en assurant une liberté de mouvement satisfaisante entre les vertèbres qui lui sont adjacentes, induise seulement de faibles sollicitations mécaniques au niveau de l'ensemble de la chaîne vertébrale.

A cet effet, elle a pour objet un dispositif de stabilisation intervertébral destiné à relier deux vertèbres voisines, caractérisé en ce qu'il comprend un implant destiné à être inséré au moins partiellement entre les corps vertébraux des deux vertèbres voisines, ledit implant étant apte à conférer auxdits deux corps vertébraux voisins au moins un degré de liberté mutuel, ledit dispositif comprenant également au moins un organe extra-discal, disposé à l'arrière de l'espace intervertébral, propre à amortir un déplacement entre lesdites vertèbres, au moins dans le sens de la flexion intervertébrale.

Dans le cas où les deux vertèbres voisines possèdent un unique degré de liberté, il s'agit d'un degré de liberté en rotation, autour d'un axe transversal du patient, correspondant aux mouvements de flexion et d'extension de ce patient. Le ou chaque organe extra-discal est propre à amortir un déplacement entre ces vertèbres voisines au moins dans le sens de la flexion intervertébrale, dans laquelle le patient se penche vers l'avant. Cette flexion intervertébrale correspond à l'extension de chaque organe extra-discal, c'est-à-dire à son allongement selon sa direction principale, qui est sensiblement la direction principale de la chaîne vertébrale, soit la verticale lorsque le patient est debout.

L'invention va être décrite ci-dessous, en référence aux dessins annexés, donnés uniquement à titre d'exemples non limitatifs et dans lesquels :
- la figure 1 est une vue schématique de côté, illustrant deux vertèbres voisines entre lesquelles est placé un dispositif de stabilisation conforme à l'invention ;
- la figure 2 est une vue de côté, à plus grande échelle, illustrant un implant appartenant au dispositif de stabilisation de la figure 1 ;
- les figures 3 à 6 sont des vues analogues à la figure 2, illustrant des variantes de réalisation de l'implant appartenant au dispositif de stabilisation de la figure 1 ;
- la figure 7 est une vue de dessus, illustrant une variante supplémentaire de l'invention ; et
- la figure 8 est une vue de derrière, illustrant les deux organes d'amortissement appartenant au dispositif de stabilisation de la figure 7 ;
- la figure 9 est une vue schématique de côté, analogue à la figure 1, illustrant une variante supplémentaire de réalisation de l'invention ;
- la figure 10 est une vue en perspective, illustrant le dispositif de la figure 9 ;
- les figures 11 à 13 sont des vues de dessus, illustrant le dispositif de la figure 9, puis deux variantes de réalisations ; et
- la figure 14 est une vue de côté illustrant une dernière variante de réalisation de l'invention.

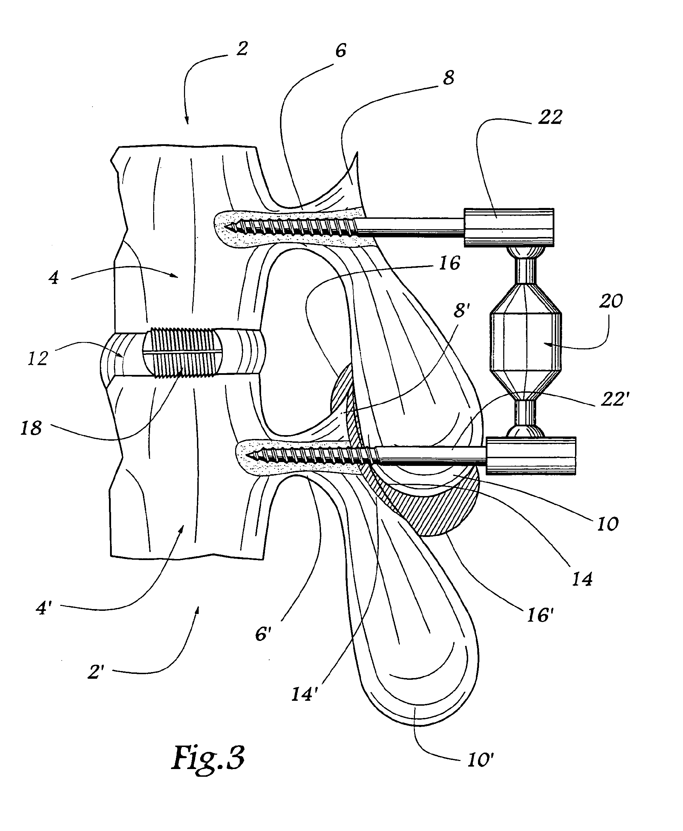

La figure 1 représente deux vertèbres respectivement supérieure 2 et inférieure 2' qui sont reliées par l'intermédiaire d'un dispositif de stabilisation conforme à l'invention. Chaque vertèbre comprend un corps vertébral 4, 4' prolongé par un pédicule 6, 6', une articulaire supérieure 8, 8' et une articulaire inférieure 10, 10'. On désigne par 12 l'espace intervertébral, par 14 et 14' les facettes articulaires en regard et par 16 et 16' les capsules articulaires.

Les deux vertèbres 2, 2' sont reliées mutuellement par l'intermédiaire d'un dispositif de stabilisation, comprenant un implant intersomatique 18, logé dans l'espace intervertébral 12, ainsi qu'un organe d'amortissement, désigné dans son ensemble par la référence 20, dont les deux extrémités sont fixées sur les vertèbres correspondantes par l'intermédiaire de vis pédiculaires 22, 22'.

L'organe d'amortissement est par exemple conforme à l'enseignement de FR-A-2 676 911, ou bien encore à celui de FR-A-2 751 864. Il peut également comprendre un ligament, conformément par exemple à l'enseignement de FR-A-2 694 182.

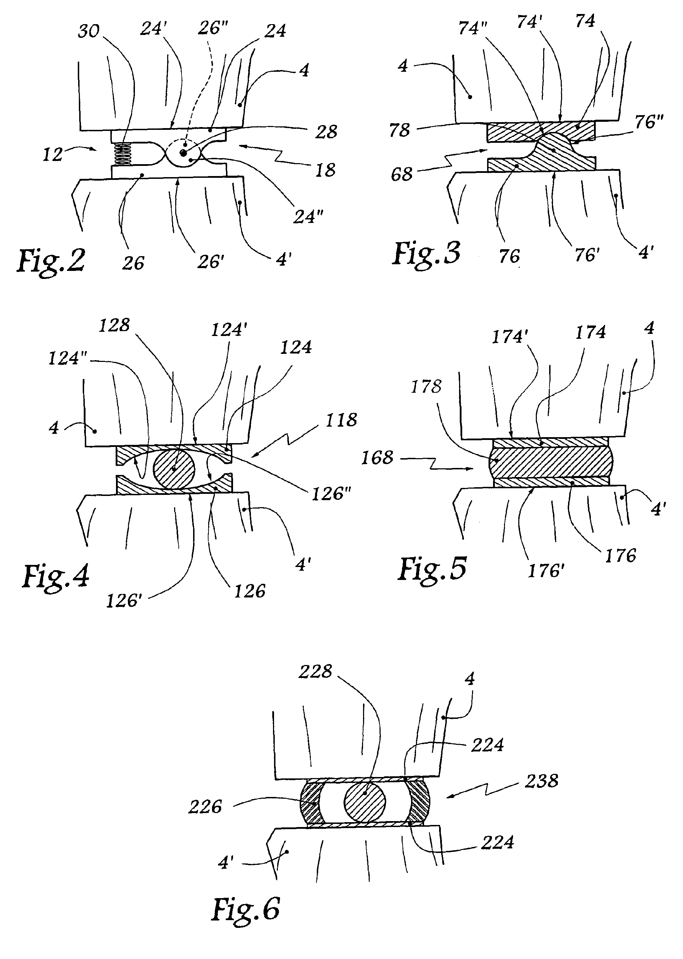

L'implant 18 est représenté de façon plus précise sur la figure 2. Sur cette dernière, comme sur les figures suivantes, le côté droit correspond à la partie postérieure de l'espace intervertébral 12, le côté gauche correspondant à la partie antérieure.

L'implant 18 comprend des éléments respectivement supérieur 24 et inférieur 26, entrant en contact avec les corps vertébraux 4, 4', par des surfaces de contact 24', 26' respectives, qui sont planes. A titre de variante, ces surfaces de contact peuvent être de formes différentes, notamment convexes.

Les éléments 24, 26 comportent des chapes respectives 24'', 26'', qui sont articulées au moyen d'un axe 28 s'étendant transversalement. Cet axe 28, qui est disposé du côté postérieur de l'espace intervertébral 12, confère un degré de liberté entre les surfaces 24' et 26', et donc entre les corps vertébraux 4, 4'. Ce degré de liberté unique est une rotation autour de l'axe 28 transversal, ou sagittal, ou à une extension ce qui correspond ainsi à une flexion du patient vers l'avant de celui-ci vers l'arrière.

Un ressort 30, travaillant en compression, est fixé aux éléments 24, 26, du côté antérieur de l'espace intervertébral 12. Ce ressort peut être remplacé par un bloc résilient, réalisé par exemple en élastomère, notamment en caoutchouc.

La figure 3 illustre un second mode de réalisation de l'implant intervertébral, désigné par la référence 68. Ce dernier comprend deux éléments respectivement supérieur 74 et inférieur 76, entrant en contact avec les corps vertébraux 4, 4' par l'intermédiaire de surfaces de contact planes 74', 76'.

L'un de ces éléments, en l'occurrence l'élément supérieur, est pourvu d'un logement sphérique 74'', formant cupule, disposé vers la partie postérieure de l'espace intervertébral 12. Ce logement 74'' coopère avec une saillie sphérique 76" de l'autre élément, à savoir l'élément inférieur 76.

Etant donné que les rayons du logement 74'' et de la saillie 76'' sont sensiblement identiques, leur coopération assure trois degrés de liberté en rotation, autour du centre fixe 78 de la saillie sphérique 76", des surfaces de contact 74', 76' et donc des corps vertébraux 4, 4'.

Il est possible de munir cet implant 68 d'un ou plusieurs ressorts, analogues à celui 30, ou d'un bloc résilient, s'étendant entre les éléments supérieur et inférieur, par exemple du côté antérieur de l'espace intervertébral.

La figure 4 illustre un troisième mode de réalisation de l'implant intervertébral, qui est désigné dans son ensemble par la référence 118. Cet implant comprend deux éléments supérieur 124 et inférieur 126, formant plateaux. Chacun de ces plateaux, qui prend appui contre un corps vertébral respectif par l'intermédiaire d'une surface plane 124', 126', est pourvu d'un renfoncement sphérique 124'', 126''. Une bille 128 qui possède un rayon de courbure sensiblement inférieur à celui des renfoncements 124", 126", est intercalée entre les plateaux 124, 126.

Cette bille 128 est libre de se déplacer au voisinage de la surface adjacente des plateaux 124, 126, ce qui confère trois degrés de liberté en rotation, aux deux corps vertébraux 4, 4' autour d'un point mobile, ainsi que deux degrés de liberté en translation autorisés par les glissements des plateaux sur cette bille. Cette dernière peut être remplacée par un organe non sphérique, par exemple ovale ou cylindrique, prenant appui contre les plateaux 124, 126 par l'intermédiaire d'une surface de contact dont le rayon de courbure est inférieur à celui des renfoncements 124", 126" précités, afin de permettre un déplacement mutuel de cet organe par rapport au plateau.

La figure 5 représente une quatrième variante de réalisation de l'implant intervertébral, désigné dans son ensemble par la référence 168. Ce dernier comprend un enrobage, formé par deux plateaux rigides 74, 176, entre lesquels est intercalée une âme élastique 178. Les plateaux 174, 176 recouvrent partiellement l'âme 178, en ce sens qu'ils sont disposés sur les bords de cette âme adjacents aux corps vertébraux. Les plateaux sont par exemple réalisés en titane alors que l'âme, qui est par exemple collée au plateau, est réalisée par exemple en silicone ou en élastomère, notamment en caoutchouc.

Les plateaux 174, 176 entrent en contact avec les corps vertébraux 4, 4' par l'intermédiaire de surfaces planes 174', 176'. Cet implant est inséré dans l'espace intervertébral par impaction, comme les implants 18, 68 et 118. Il est également envisageable que la distance séparant les surfaces de contact 174', 176', qui correspond à la dimension verticale de l'implant posé, augmente vers sa partie antérieure, dans la position anatomique verticale du repos du patient.

La figure 6 illustre un cinquième mode de réalisation de l'implant intervertébral, désigné dans son ensemble par la référence 218. Ce dernier comprend une bille rigide 228 entourée par un anneau périphérique 226, d'axe principal parallèle à l'axe principal de la colonne vertébrale, cet anneau étant réalisé en un matériau élastique tel que du caoutchouc. Les extrémités latérales de l'anneau sont solidaires de plaques 224 qui entrent en contact avec les corps vertébraux respectifs 4, 4'.

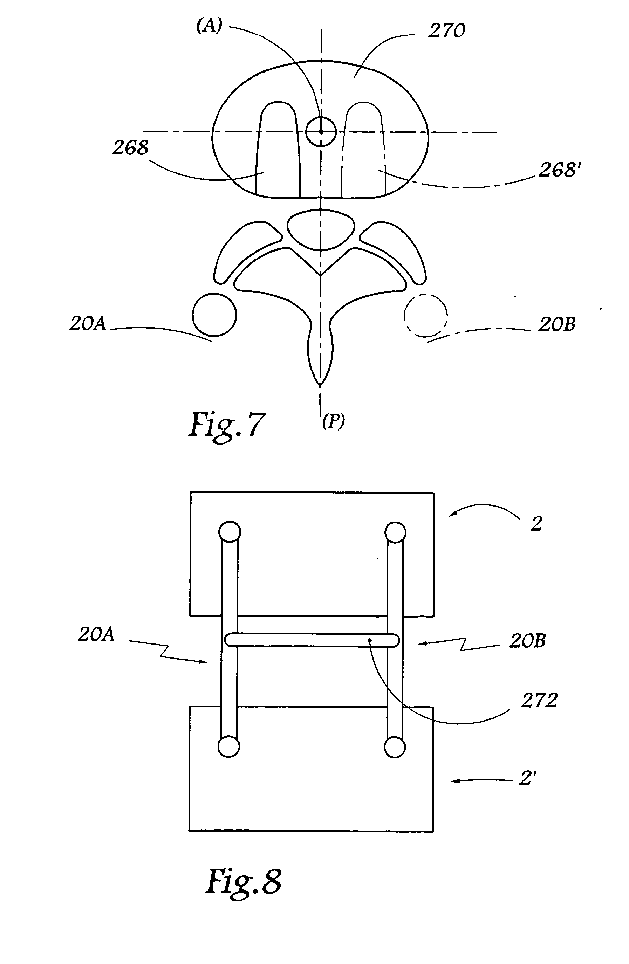

Les implants 18, 68, 118, 168, 218 décrits ci-dessus sont des prothèses totales de disque. Il est également possible de faire appel à un implant 268, illustré sur la figure 7, qui est une prothèse partielle de disque. Cette dernière 268, qui est insérée dans le disque 270, est disposée de façon décalée, par rapport à l'axe principal A de la chaîne vertébrale qui, lorsque le patient se trouve en position debout, est un axe vertical passant par le plan médian P s'étendant d'arrière en avant du patient. Cette prothèse peut être insérée par vissage ou impactage dans l'espace intervertébral.

Cette prothèse partielle 268 est associée à un organe d'amortissement 20A, qui est disposée, de façon décalée, du même côté de l'axe A que la prothèse partielle 268.

On peut associer à la prothèse partielle 268, une prothèse supplémentaire 268', située de l'autre côté de l'axe A. Cette prothèse partielle 268', qui est représentée en traits mixtes, peut être analogue à la prothèse partielle 268 étant entendu qu'il est possible de conférer à ces deux prothèses partielles 268, 268' des hauteurs différentes, de manière à compenser un éventuel effondrement du disque se produisant de façon asymétrique, en vue de derrière. La prothèse partielle 268' est associée à un organe d'amortissement supplémentaire 20B, représenté en traits mixtes, qui est prévu du même côté de l'axe A que la prothèse partielle 268'.

La figure 8 représente les deux organes d'amortissement 20A, 20B disposés de part et d'autre des articulaires 8, 10. Ces organes d'amortissement présentent une partie métallique et sont par exemple conformes à l'enseignement de FR-A-2 751 864. Ils sont avantageusement reliés l'un à l'autre au moyen d'une tige transversale 272, s'étendant sensiblement horizontalement. La liaison entre chaque organe 20, 20A et la tige 272 est rigide, et fait par exemple intervenir une soudure. Elle est avantageusement réalisée au niveau de la partie médiane de ces organes d'amortissement.

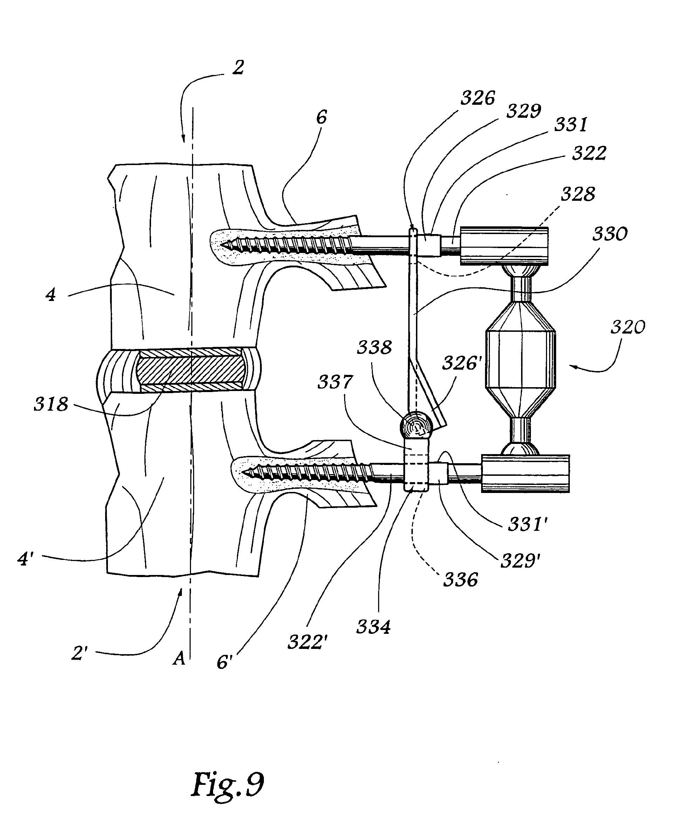

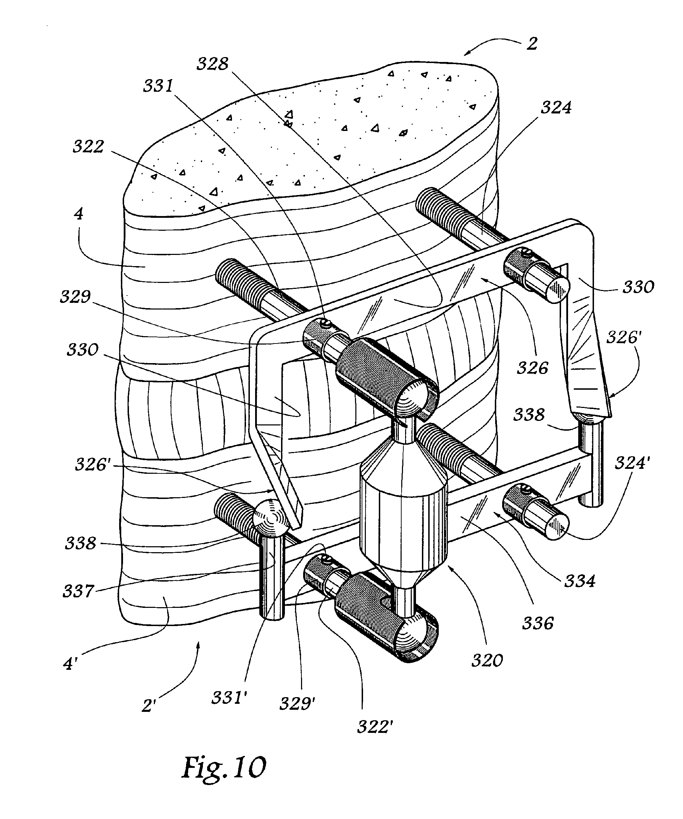

Les figures 9 à 11 illustrent une variante supplémentaire de l'invention, dans laquelle il est prévu deux vis pédiculaires supérieures 322, 324, disposées de part et d'autre de l'axe principal de la colonne vertébrale, ainsi que deux vis pédiculaires inférieures 322', 324'. Le dispositif de stabilisation comprend un implant 318, par exemple analogue à celui 168, ainsi qu'un organe extra-discal 320, qui est analogue à celui 20.

Ce dispositif de stabilisation comprend également un élément de butée supérieur 326, comportant une branche horizontale 328 ainsi que deux branches verticales 330. Cette branche 328 est creusée de deux ouvertures circulaires destinées au passage de la tige des vis pédiculaires supérieures 322, 324. Les parois de chaque ouverture sont prolongées par un fourreau axial 329, recouvrant une partie de la vis. Ce fourreau, qui peut être venu de matière avec la branche 328, reçoit une vis d'arrêt 331 apte à immobiliser de façon sélective l'élément de butée par rapport à la vis pédiculaire, selon une translation parallèle à l'axe principal de cette dernière.

Ce dispositif comprend également un élément de butée inférieur 334, comportant une branche horizontale 336 prolongée, à ses deux extrémités, par des tiges 337 pourvues de sphères 338. Cet élément inférieur est creusé de deux ouvertures, destinées au passage de la tige des deux vis pédiculaires inférieures 322', 324'. De façon analogue à ce qui a été décrit ci-dessus pour l'élément supérieur, chaque ouverture est pourvue d'un fourreau axial 329', muni d'une vis 331'.

Par ailleurs, en variante, au moins une des ouvertures peut être une lumière oblongue. Ceci permet ainsi d'adapter les dimensions des éléments de butée à différents espacements des vis pédiculaires. Les branches horizontales 328 et 336 peuvent présenter des longueurs variables, en étant par exemple télescopiques.

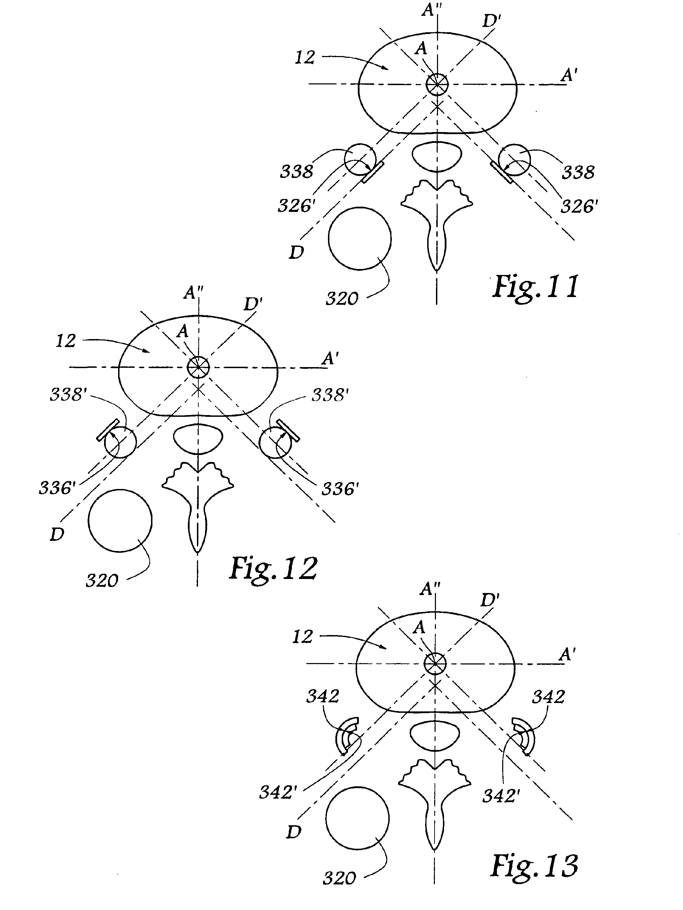

Chaque branche verticale 330 est repliée, de sorte que son extrémité possède une surface plane 326' s'étendant de façon oblique. Ceci signifie que cette extrémité n'est ni parallèle à l'axe transversal médian A', s'étendant de la droite vers la gauche du patient, ni parallèle à l'axe sagittal médian A", s'étendant d'arrière en avant du patient (figure 11). L'axe principal D de cette surface plane 326' est parallèle à une droite D' passant par l'intersection de ces deux axes A' et A", notamment une bissectrice.

Chaque surface 326' coopère avec une sphère 338 correspondante, selon un contact sensiblement ponctuel. De la sorte, deux rotations autour des axes A' et A'' sont autorisées entre les éléments de butée supérieur et inférieur et, ce faisant, entre les deux vertèbres 2 et 2'. En revanche, la rotation autour de l'axe vertical A est interdite entre ces deux vertèbres.

Par ailleurs, une mise en translation mutuelle des deux vertèbres 2, 2', selon l'axe sagittal A", est autorisée dans un unique sens. Ainsi, la vertèbre supérieure ne peut se déplacer vers l'avant, par rapport à la vertèbre inférieure, mais en revanche est libre de se déplacer vers l'arrière par rapport à cette vertèbre inférieure.

En outre, toute translation mutuelle des deux vertèbres 2, 2' est interdite, dans les deux sens, selon l'axe transversal A'. Enfin, une translation mutuelle entre ces deux vertèbres est autorisée, dans les deux sens, selon l'axe vertical A.

D'autres agencements peuvent être envisagés. Ainsi, l'élément de butée supérieur peut être muni d'au moins une sphère 338', coopérant avec une branche verticale, terminée par une surface plane oblique 336', s'étendant à partir de la branche horizontale 336 de l'élément inférieur (figure 12). Il peut être fait appel à la coopération de deux surfaces d'appui sphériques adjacentes 342, 342', dont chacune appartient à un élément de butée respectif (figure 13).

A titre de variante supplémentaire, au moins une des branches verticales 330 peut, au moins partiellement, être réalisée en un matériau élastique, dont l'élasticité autorise un contact permanent entre chaque branche 330 et une sphère correspondante 338. Il est également envisageable de réaliser au moins une branche verticale en deux parties, possédant un certain débattement mutuel en rotation, autour de l'axe principal de la branche. Cette possibilité de débattement peut être provisoire, pour la mise en place des deux éléments de butée, ou permanente afin d'assurer à chaque instant une adaptation angulaire entre la branche et la sphère.

Il est possible de prévoir une unique branche verticale 330, coopérant avec une unique sphère 338, notamment dans le cas où une partie de l'articulation postérieure naturelle n'a pas été détruite.

Etant donné que chaque élément supérieur ou inférieur est monté sur deux vis pédiculaires à la fois, ceci permet d'éviter toute désolidarisation de ces vis par rapport aux corps vertébraux, une fois celles-ci mises en place.

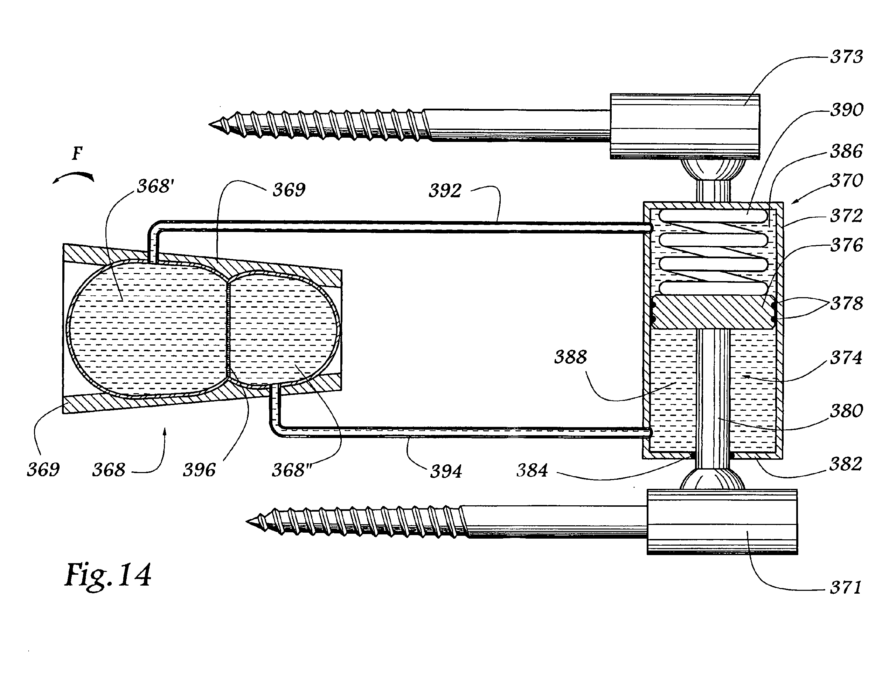

La figure 14 illustre une variante supplémentaire de réalisation de l'invention. Le dispositif qui y est décrit comprend un implant 368, destiné à être inséré au moins partiellement dans l'espace intervertébral. Cet implant comprend deux chambres respectivement avant 368' et arrière 368'', entourées par deux coquilles 369. Chacune de ces dernières, qui présente une section transversale sensiblement en arc de cercle, est réalisée en un matériau rigide, tel que du titane. Ces coquilles 369, qui sont destinées à entrer en contact avec les corps vertébraux 4, 4', sont assujetties aux chambres, par exemple par collage.

Ces deux chambres 368, 368', qui sont séparées par une membrane 396, éventuellement poreuse, sont remplies d'un fluide d'amortissement. Ce dernier comprend au moins un liquide, tel que de l'eau ou de l'huile. Il peut également contenir de l'air, ou encore un corps hydrophile, tel de l'hydrogel.

Le dispositif de stabilisation illustré sur cette figure 11 comprend également un organe d'amortissement 370, disposé à l'arrière de l'espace intervertébral. Cet organe comprend une cuve rigide 372 à l'intérieur de laquelle est disposé un piston 374, qui comprend une tête 376, formant extrémité supérieure, dont les dimensions transversales sont voisines de celles de la cuve. Un joint torique d'étanchéité 378 est monté entre les parois en regard de la tête et de la cuve.

La tête 376 du piston s'étend à partir d'une tige 380 verticale qui traverse, de façon étanche, la paroi inférieure 382 de la cuve 372, avec interposition d'un joint torique 384. L'extrémité inférieure de la tige 380, opposée à la tête 376, est montée à rotule sur la tête de la vis inférieure 371.

La tête du piston délimite des chambres respectivement supérieure 386 et inférieure 388, appartenant à la cuve 372. La chambre supérieure reçoit un ressort 390, travaillant en compression, qui s'étend verticalement entre la paroi supérieure de la cuve et la paroi en regard de la tête. Ce ressort permet le rappel du piston dans sa position basse, qui correspond à une posture lordosée du patient, physiologiquement avantageuse.

La chambre avant de l'implant 368 est mise en communication fluidique avec la chambre supérieure de l'organe 370, par l'intermédiaire d'un conduit 392, alors que la chambre arrière de l'implant est mise en communication fluidique avec la chambre inférieure 388 par l'intermédiaire d'un conduit supplémentaire 394. De la sorte, lorsque le patient se penche vers l'avant, selon la flèche F, du fluide est chassé de la chambre avant en direction de la chambre supérieure, ce qui contribue à faire descendre le piston 374 dans la cuve, à l'opposé de la vis supérieure 373. Cette remontée induit un déplacement de fluide, par le conduit 394, de la chambre inférieure vers la chambre arrière. Ce mouvement de flexion est donc amorti par ces différents écoulements de fluide.

L'invention n'est pas limitée aux exemples décrits et représentés.

L'implant appartenant au dispositif de stabilisation de l'invention peut être partiel ou total. Dans le cas où il s'agit d'un implant partiel, plusieurs implants de ce type peuvent être disposés entre deux mêmes vertèbres. Cet implant peut être mis en place, soit par voie antérieure, soit par voie postérieure.

Par ailleurs, on peut prévoir d'utiliser une bille unique, analogue à celle 228, qui confère trois degrés de liberté en rotation, ainsi que deux degrés de liberté en translation aux corps vertébraux 4, 4'. Il est également possible de réaliser l'implant intervertébral sous forme d'une enveloppe renfermant un gel hydrophile ou de l'eau, cet implant constituant une prothèse de nucleus.

Il est également possible de fixer l'implant sur la paroi verticale des corps vertébraux, par exemple par vissage, conformément à l'enseignement de EP-A-0 346 269, cet implant étant alors inséré seulement partiellement entre les deux corps vertébraux.

L'implant intervertébral peut renfermer un fluide d'amortissement, et être conforme à l'un des modes de réalisation décrits dans la demande de brevet français, déposée le 29 décembre 1999, sous le numéro 99 16662. Cet implant intervertébral fluidique est susceptible de coopérer avec un organe extra-discal de type mécanique, par exemple analogue à celui 20.

Par ailleurs, l'organe extra-discal peut également renfermer un tel fluide d'amortissement, et être conforme à l'un des modes de réalisation décrits dans la demande de brevet français ci-dessus mentionnée. Cet organe extra-discal fluidique est susceptible de coopérer avec un implant intervertébral mécanique, tel que par exemple celui 18.

L'invention permet de réaliser les objectifs précédemment mentionnés.

En cas de pathologie dégénérative du disque intervertébral, s'étendant aux nerfs qui lui sont adjacents, il est nécessaire pour le chirurgien de libérer la racine nerveuse ainsi comprimée. A cet effet, l'opération correspondante induit une destruction au moins partielle de l'articulation intervertébrale postérieure.

Le dispositif de l'invention permet de restaurer la stabilité postérieure, qui avait été sensiblement diminuée du fait de la chirurgie.

L'implant intervertébral permet de restaurer la hauteur du disque et de rappeler le mouvement intervertébral naturel, étant donné qu'il confère au moins un degré de liberté aux corps vertébraux en regard. Par ailleurs, l'organe d'amortissement extra-discal garantit une composante supplémentaire à cette stabilisation postérieure.

Le fait de combiner cet implant intervertébral et cet organe extra-discal permet de réaliser ces deux éléments de manière simple et fiable. Il est ainsi possible de répartir, entre ces deux éléments, les différentes fonctions mécaniques qui sont nécessaires, en vue d'assurer une stabilité intervertébrale satisfaisante. Ceci permet donc de réduire les contraintes mécaniques exercées sur chacun de ces deux éléments, de sorte que ces derniers sont soumis à une usure restreinte. Ceci en allonge donc la durée de vie de façon correspondante.

Le dispositif de stabilisation de l'invention garantit ainsi que le mouvement relatif des deux vertèbres qu'il relie est suffisamment proche du mouvement autorisé par un disque vertébral naturel, pour que n'apparaisse pas de dysfonctionnement majeur au niveau de l'ensemble de la chaîne vertébrale.

L'emploi de deux organes d'amortissement extra-discaux, disposés de part et d'autres d'un axe principal de la colonne vertébrale est avantageux. En effet, il assure une composante supplémentaire d'amortissement, lorsque le patient se penche sur les côtés.

Prévoir des moyens de liaison entre ces deux organes d'amortissement est avantageux, dans la mesure où cela assure une réduction sensible du cisaillement horizontal intervertébral, comme en cas de rupture ou d'absence d'un ou de deux massifs articulaires postérieurs.

Réaliser l'implant intervertébral sous la forme d'au moins une prothèse partielle est avantageux. En effet, de telles prothèses, du fait de leur dimensions, peuvent être introduites depuis l'arrière du patient, de sorte qu'il peut être fait appel à une unique opération, au cours de laquelle ces prothèses sont implantées en même temps que l'organe d'amortissement.

Prévoir une unique prothèse, associée à un unique organe d'amortissement extra-discal, tous deux décalés d'un même côté de l'axe principal de la chaîne vertébrale, permet de remédier à des effondrements asymétriques de l'espace intervertébral, vu de derrière. De tels effondrements asymétriques peuvent également être palliés en faisant appel à deux prothèses de hauteurs différentes, disposées de part et d'autre de l'axe principal de la chaîne vertébrale.

## Revendications

1. Dispositif de stabilisation intervertébral destiné à relier deux vertèbres voisines (2, 2'), le dispositif comprenant un implant (18 ; 68 ; 118 ; 168 ; 218 ; 268 ; 318 ; 368) destiné à être inséré au moins partiellement entre les corps vertébraux (4, 4') des deux vertèbres voisines, ledit implant (18 ; 68 ; 118 ; 168 ; 218 ; 268 ; 318 ; 368) étant apte à conférer auxdits deux corps vertébraux voisins (4, 4') au moins un degré de liberté mutuel, ledit dispositif comprenant également au moins un organe extra-discal (20 ; 320 ; 370), apte à être disposé à l'arrière de l'espace intervertébral (12), propre à amortir un déplacement entre lesdites vertèbres (2, 2'), au moins dans le sens de la flexion intervertébrale.

2. Dispositif selon la revendication 1, **caractérisé en ce que** ledit implant (18 ; 68) est apte à conférer aux deux corps vertébraux (4, 4') au moins un degré de liberté en rotation, autour d'un pivot fixe (28 ; 78) dudit implant.

3. Dispositif selon la revendication 2, **caractérisé en ce qu'**il est prévu des moyens supplémentaires d'amortissement (30) au moins dans le sens de la flexion intervertébrale, disposés vers la partie antérieure dudit implant (18 ; 68).

4. Dispositif selon la revendication 1, **caractérisé en ce que** ledit implant (118) est apte à conférer aux deux corps vertébraux (4, 4') au moins un degré de liberté en rotation, autour d'un pivot mobile (128 ; 228) dudit implant.

5. Dispositif selon la revendication 1, **caractérisé en ce que** ledit implant (168) comprend une âme élastique (178), notamment réalisée en un polymère de silicone ou un élastomère, partiellement recouverte par un enrobage (174, 176) réalisé en un matériau rigide.

6. Dispositif selon l'une quelconque des revendications précédentes, **caractérisé en ce qu'**il est prévu deux organes d'amortissement extra-discaux (20A, 20B) disposés de part et d'autre d'un axe principal (A) de la chaîne vertébrale.

7. Dispositif selon la revendication 6, **caractérisé en ce qu'**il est prévu en outre des moyens de solidarisation (272) des organes d'amortissement (20A, 20B) entre eux.

8. Dispositif selon l'une des revendications précédentes, **caractérisé en ce que** ledit implant comprend au moins une prothèse partielle (268, 268') de disque.

9. Dispositif selon la revendication 8, **caractérisé en ce que** ledit implant intervertébral comprend une unique prothèse partielle (268), associée à un unique organe (20A) d'amortissement extra-discal, tous deux étant décalés d'un même côté par rapport à l'axe principal (A) de la chaîne vertébrale.

10. Dispositif selon la revendication 9, **caractérisé en ce que** ledit implant intervertébral comprend deux prothèses partielles (268, 268') disposées de part et d'autre dudit axe principal (A), ainsi que deux organes (20A, 20B) d'amortissement extra-discaux, disposés de part et d'autre dudit axe (A).

11. Dispositif selon l'une des revendications précédentes, **caractérisé en ce qu'**il comprend en outre un élément de butée supérieur (326), solidaire d'une vertèbre supérieure, ainsi qu'un élément de butée inférieur (334), solidaire d'une vertèbre inférieure, ces éléments de butée extra-discaux possédant des surfaces d'appui mutuelles (326', 338 ; 336', 338' ; 342, 342') aptes à autoriser une rotation mutuelle desdites vertèbres supérieure (2) et inférieure (2') autour d'axes transversal (A') et sagittal (A'') du patient, ainsi qu'à empêcher une rotation mutuelle de ces deux vertèbres autour d'un axe vertical (A), ces surfaces d'appui étant en outre aptes à autoriser une translation mutuelle de ces vertèbres dans un unique sens selon l'axe sagittal (A"), à autoriser une translation entre ces deux vertèbres dans les deux sens selon l'axe vertical (A), et à interdire une translation entre ces deux vertèbres dans les deux sens selon l'axe transversal (A').

12. Dispositif selon la revendication 11, **caractérisé en ce que** l'un desdits éléments de butée comprend deux surfaces d'appui planes (326'), disposées de part et d'autre de l'axe vertical (A), ces deux surfaces s'étendant de façon oblique et coopérant avec deux sphères (338) dont est pourvu l'autre desdits éléments.

13. Dispositif selon les revendications 11 ou 12, **caractérisé en ce que** chaque élément de butée (326, 334) est solidaire de deux vis pédiculaires, respectivement supérieures (322, 324) et inférieures (322', 324').

14. Dispositif selon la revendication 13, **caractérisé en ce qu'**il est prévu des moyens (331, 331') permettant de solidariser en translation, de façon sélective, chaque élément de butée avec au moins une vis pédiculaire.

15. Dispositif selon l'une quelconque des revendications précédentes, **caractérisé en ce que** ledit implant (368) comprend au moins une chambre (368', 368'') et **en ce que** ledit organe extra-discal (370) comprend au moins une chambre (386, 388), lesdites au moins deux chambres renfermant un fluide, et **en ce que** sont prévus des moyens (392, 394) de mise en communication fluidique de ces chambres.

16. Dispositif selon la revendication 15, **caractérisé en ce que** les moyens de mise en communication comprennent au moins un conduit (392, 394) s'étendant entre ces chambres.

17. Dispositif selon la revendication 15, **caractérisé en ce qu'**un premier conduit assure la communication entre les chambres dans un premier sens, alors qu'un second conduit assure cette communication dans un second sens, opposé au premier, les sections de passage des premier et second conduits étant différentes.

18. Dispositif selon l'une des revendications 15 à 17, **caractérisé en ce que** l'implant (368) et/ou l'organe extra-discal comprend au moins deux chambres (368', 368'') et **en ce qu'**un élément poreux (396) sépare lesdites chambres.

19. Dispositif selon la revendication 18, **caractérisé en ce que** ledit organe poreux est mobile par rapport à ces chambres.

20. Dispositif selon l'une des revendications 15 à 19, **caractérisé en ce qu'**il est prévu au moins un piston (374), séparant deux chambres adjacentes.

21. Dispositif selon l'une des revendications 15 à 20, **caractérisé en ce qu'**au moins une chambre est pourvue d'une paroi déformable.

## Claims

1. A device for inter-vertebral stabilisation, intended to connect two adjacent vertebrae (2, 2'), the device comprising an implant (18; 68; 118; 168; 218; 268; 318; 368) which is intended to be at least partly inserted between the vertebral bodies (4, 4') of two adjacent vertebrae, the said implant (18; 68; 118; 168; 218; 268; 318; 368) being capable of giving the said two adjacent vertebral bodies (4, 4') at least one degree of mutual freedom, the said device also comprising at least one extra-disc member (20; 320; 370) capable of being arranged to the rear of the inter-vertebral space (12) and suitable for damping displacement between the said vertebrae (2, 2') at least in the direction of inter-vertebral flexion.

2. A device according to Claim 1, **characterised in that** the said implant (18; 68) is capable of giving the two vertebral bodies (4, 4') at least one degree of freedom in rotation about a fixed pivot point (28; 78) of the said implant.

3. A device according to Claim 2, **characterised in that** it is provided with additional means (30) of damping at least in the direction of inter-vertebral flexion, these means being arranged towards the anterior part of the said implant (18; 68).

4. A device according to Claim 1, **characterised in that** the said implant (118) is capable of giving the two vertebral bodies (4, 4') at least one degree of freedom in rotation about a movable pivot point (128; 228) of the said implant.

5. A device according to Claim 1, **characterised in that** the said implant (168) comprises a resilient core (178) made in particular of a silicone polymer or an elastomer and partly covered in a coating (174, 176) made of a rigid material.

6. A device according to any one of the preceding claims, **characterised in that** it is provided with two extra-disc damping members (20A, 20B) which are arranged on either side of a main axis (A) of the vertebral column.

7. A device according to Claim 6, **characterised in that** means (272) are furthermore provided for fixing the damping members (20A, 20B) to one another.

8. A device according to one of the preceding claims, **characterised in that** the said implant comprises at least one partial disc prosthesis (268, 268').

9. A device according to Claim 8, **characterised in that** the said inter-vertebral implant comprises a single partial prosthesis (268) associated with a single extra-disc damping member (20A), both these being offset to the same side in relation to the principal axis (A) of the vertebral column.

10. A device according to Claim 9, **characterised in that** the said inter-vertebral implant comprises two partial prostheses (268, 268') arranged on either side of the said principal axis (A), and two extra-disc damping members (20A, 20B) which are arranged on either side of the said axis (A).

11. A device according to one of the preceding claims, **characterised in that** it furthermore comprises an upper stop element (326) which is fixed to an upper vertebra, and a lower stop element (334) which is fixed to a lower vertebra, these extra-disc stop elements having mutual bearing surfaces (326', 338; 336', 338'; 342, 342') which are capable of enabling mutual rotation of the said upper vertebra (2) and lower vertebra (2') about a transverse axis (A') and a sagittal axis (A'') of the patient, and of preventing mutual rotation of these two vertebrae about a vertical axis (A), these bearing surfaces moreover being capable of enabling a mutual translational movement of these vertebrae in a single direction along the sagittal axis (A"), enabling a translational movement between these two vertebrae in the two directions along the vertical axis (A), and preventing a translational movement between these two vertebrae in the two directions along the transverse axis (A').

12. A device according to Claim 11, **characterised in that** one of the said stop elements comprises two planar bearing surfaces (326') which are arranged on either side of the vertical axis (A), these two surfaces extending obliquely and cooperating with two spheres (338) with which the other of the said elements is provided.

13. A device according to Claim 11 or 12, **characterised in that** each stop element (326, 334) is fixed to two pedicle screws, upper pedicle screws (322, 324) and lower pedicle screws (322', 324') respectively.

14. A device according to Claim 13, **characterised in that** it is provided with means (331, 331') allowing each stop element to be selectively fixed as regards translational movement to at least one pedicle screw.

15. A device according to any one of the preceding claims, **characterised in that** the said implant (368) comprises at least one chamber (368', 368"), and **in that** the said extra-disc member (370) comprises at least one chamber (386, 388), the said at least two chambers enclosing a fluid, and **in that** means (392, 394) of providing fluid communication between these chambers are provided.

16. A device according to Claim 15, **characterised in that** the means of providing communication comprise at least one duct (392, 394) extending between these chambers.

17. A device according to Claim 15, **characterised in that** a first duct ensures communication between the chambers in a first direction, while a second duct ensures this communication in a second direction opposed to the first, the cross-sections of passage of the first and second ducts being different.

18. A device according to one of Claims 15 to 17, **characterised in that** the implant (368) and/or the extra-disc member comprises at lest two chambers (368', 368"), and **in that** a porous element (396) separates the said chambers.

19. A device according to Claim 18, **characterised in that** the said porous member is movable in relation to these chambers.

20. A device according to one of Claims 15 to 19, **characterised in that** at least one piston (374) separating two adjacent chambers is provided.

21. A device according to one of Claims 15 to 20, **characterised in that** at lest one chamber is provided with a deformable wall.

## Patentansprüche

1. Vorrichtung zur Zwischenwirbelstabilisierung, mit der zwei benachbarte Wirbel (2, 2') verbindbar sind, wobei die Vorrichtung ein Implantat (18; 68; 118; 168; 218; 268; 318; 368) aufweist, das zumindest teilweise in die Wirbelkörper (4, 4') der beiden benachbarten Wirbel einführbar ist, wobei dieses Implantat (18; 68; 118; 168; 218; 268; 318; 368) fähig ist, den beiden benachbarten Wirbelkörpern 4, 4') zumindest einen gegenseitigen Freiheitsgrad zu verleihen, wobei diese Vorrichtung auch zumindest ein extradiscales Organ (20; 320; 370) aufweist, das fähig ist, im hinteren Bereich des Zwischenwirbelraumes (12) angeordnet zu werden, das geeignet ist, eine Verlagerung zwischen diesen Wirbeln (2, 2') zumindest im Sinne der Zwischenwirbelbiegung zu dämpfen.

2. Vorrichtung nach Anspruch 1, **dadurch gekennzeichnet, dass** das Implantat (18; 68) geeignet ist, den beiden Wirbelkörpern (4, 4') zumindest einen Rotationsfreiheitsgrad um eine feste Drehachse (28; 78) des Implantats zu verleihen.

3. Vorrichtung nach Anspruch 2, **dadurch gekennzeichnet, dass** zusätzliche Dämpfungseinrichtungen (30) zumindest im Sinne der Zwischenwirbelbiegung vorgesehen sind, die im vorderen Bereich des Implantes (18; 68) gelegen sind.

4. Vorrichtung nach Anspruch 1, **dadurch gekennzeichnet, dass** das Implantat (118) fähig ist, den beiden Wirbelkörpern (4, 4') zumindest einen Rotationsfreiheitsgrad um eine bewegliche Drehachse (128, 228) des Implantes zu verleihen.

5. Vorrichtung nach Anspruch 1, **dadurch gekennzeichnet, dass** das Implantat (168) einen elastischen Kern (178) aufweist, der insbesondere aus einem Silikonpolymer oder einem Elastomer hergestellt und teilweise durch eine Ummantelung (74, 176) aus einem festen Material bedeckt ist.

6. Vorrichtung nach einem der vorhergehenden Ansprüche, **dadurch gekennzeichnet, dass** zwei extradiscale Dämpfungselemente (20A, 20B) vorgesehen sind, die auf beiden Seiten einer Hauptachse (A) der Wirbelsäule gelegen sind.

7. Vorrichtung nach Anspruch 6, **dadurch gekennzeichnet, dass** des Weiteren Versteifungseinrichtungen (272) zwischen den Dämpfungselementen (20A, 20B) vorgesehen sind.

8. Vorrichtung nach einem der vorhergehenden Ansprüche, **dadurch gekennzeichnet, dass** das Implantat zumindest eine Teilprothese (268, 268') einer Bandscheibe aufweist.

9. Vorrichtung nach Anspruch 8, **dadurch gekennzeichnet, dass** das Zwischenwirbelimplantat eine einzige Teilprothese (268) aufweist, die einem einzigen Element (20A) zur extradiscalen Dämpfung zugeordnet ist, wobei alle beide auf die gleiche Seite in Bezug zu der Hauptachse (A) der Wirbelsäule geneigt sind.

10. Vorrichtung nach Anspruch 9, **dadurch gekennzeichnet, dass** das Zwischenwirbelimplantat zwei Teilprothesen (268, 268'), die auf beiden Seiten der Hauptachse (A) gelegen sind, und ebenso zwei extradiscale Dämpfungselemente (20A, 20B) aufweist, die auf beiden Seiten dieser Achse (A) gelegen sind.

11. Vorrichtung nach einem der vorhergehenden Ansprüche, **dadurch gekennzeichnet, dass** sie darüber hinaus ein oberes Anschlagelement (326), das fest mit einem oberen Wirbel verbunden ist, und ebenso ein unteres Anschlagelement (334) aufweist, das fest mit einem unteren Wirbel verbunden ist, wobei diese extradiscalen Anschlagelemente gegenseitige Anlageflächen (326', 338; 336', 338'; 342, 342') aufweisen, die geeignet sind, eine gegenseitige Drehung des oberen Wirbels (2) gegenüber dem unteren Wirbel (2') um eine transversale Achse (A') und eine sagittale Achse (A") des Patienten zu erlauben und gleichzeitig eine gegenseitige Rotation dieser zwei Wirbel um eine vertikale Achse (A) zu verhindern, wobei ferner die Anlageflächen zusätzlich fähig sind, eine gegenseitige Translation dieser Wirbel in einer Richtung entlang der sagittalen Achse zu erlauben, eine Translation zwischen den beiden Wirbeln in den beiden Richtungen längs der vertikalen Achse (A) zu erlauben und eine Translation zwischen den beiden Wirbeln in den beiden Richtungen längs der transversalen Achse (A') zu unterbinden.

12. Vorrichtung nach Anspruch 11, **dadurch gekennzeichnet, dass** eines dieser Anschlagelemente zwei ebene Anlageflächen (326') aufweist, die auf beiden Seiten der vertikalen Achse (A) gelegen sind, wobei diese Flächen sich schräg erstrecken und mit zwei Kugeln (338) zusammen wirken, die auf dem zweiten dieser Elemente vorgesehen sind.

13. Vorrichtung nach den Ansprüchen 11 oder 12, **dadurch gekennzeichnet, dass** jedes Anschlagelement (326, 334) mit zwei Schraubbolzen, nämlich oberen (322, 324) beziehungsweise unteren (322', 324') Schraubbolzen befestigt ist.

14. Vorrichtung nach Anspruch 13, **dadurch gekennzeichnet, dass** Einrichtungen (331, 331') vorgesehen sind, die es erlauben, jedes Anschlagelement in wählbarer Art in der Translationsrichtung mit Hilfe zumindest eines Schraubbolzens zu fixieren.

15. Vorrichtung nach einem der vorhergehenden Ansprüche, **dadurch gekennzeichnet, dass** das Implantat (368) zumindest eine Kammer (368', 368") aufweist, und dass das extradiscale Element (370) zumindest eine Kammer (386, 388) aufweist, wobei die zumindest zwei Kammern ein Fluid aufnehmen, und dass Einrichtungen (392, 394) vorgesehen sind, um eine Fluidkommunikation zwischen diesen Kammern einzurichten.

16. Vorrichtung nach Anspruch 15, **dadurch gekennzeichnet, dass** die Kommunikationseinrichtungen zumindest eine Leitung (392, 394) aufweist, die sich zwischen diesen Kammern erstreckt.

17. Vorrichtung nach Anspruch 15, **dadurch gekennzeichnet, dass** eine erste Leitung die Kommunikation zwischen den Kammern in einer ersten Richtung gewährleistet, dass eine zweite Leitung diese Kommunikation in einer zweiten, der ersten Richtung entgegen gesetzten Richtung sicherstellt, wobei die Abschnitte der Passage der ersten und der zweiten Leitung unterschiedlich sind.

18. Vorrichtung nach einem der Ansprüche 15 bis 17, **dadurch gekennzeichnet, dass** das Implantat (368) und/oder das extradiscale Element zumindest zwei Kammern (368', 368") aufweist, und dass ein poröses Element (396) die Kammern voneinander trennt.

19. Vorrichtung nach Anspruch 18, **dadurch gekennzeichnet, dass** das poröse Element in Bezug auf die Kammern beweglich ist.

20. Vorrichtung nach einem der Ansprüche 15 bis 19, **dadurch gekennzeichnet, dass** zumindest ein Kolben (374) vorgesehen ist, der die beiden benachbarten Kammern trennt.

21. Vorrichtung nach einem der Ansprüche 15 bis 20, **dadurch gekennzeichnet, dass** zumindest eine Kammer mit einer deformierbaren Wand vorgesehen ist.
